# EUROPÄISCHE PATENTANMELDUNG

(11) **EP 1 172 103 A2**
(43) Veröffentlichungstag der Anmeldung: **16.01.2002**
(21) Anmeldenummer: 01116804.4
(22) Anmeldetag: 10.07.2001
(51) Int. Cl.: A61K 31/325, A61P 33/00, A61P 31/12, A61P 31/04

(54) **Wirkstoffkombination aus 3-lodo-2-propinyl-butylcarbamat und einem oder mehreren 4-Hydroxybenzoesäureestern und Zubereitungen, solche Wirkstoffkombinationen enthaltend**

(30) Priorität: 13.07.2000 DE 10034137
(71) Anmelder: Beiersdorf AG, 20245 Hamburg (DE)
(72) Erfinder: Nielsen, Jens, 24558 Henstedt-Ulzburg (DE); Fänger, Sabine, 20255 Hamburg (DE); Kröpke, Rainer, 22869 Schenefeld (DE)

(57) **Zusammenfassung**

Wirkstoffkombinationen aus 3-lodo-2-propinyl-butylcarbamat und einem oder mehreren 4-Hydroxybenzoesäureestern sowie die Verwendung von Wirkstoffkombinationen aus 3-lodo-2-propinyl-butylcarbamat und einem oder mehreren 4-Hydroxybenzoesäureester als antibakterielle, antimycotische oder antivirale Wirkstoffe.

## Beschreibung

Die vorliegende Erfindung betrifft Wirkstoffkombinationen aus 3-lodo-2-propinyl-butylcarbamat und einem oder mehreren 4-Hydroxybenzoesäureestem sowie die Verwendung solcher Wirkstoffkombinationen als gegen Bakterien, Mycota und Viren wirksame Substanzen. In besonderen Ausführungsformen betrifft die vorliegende Erfindung Zubereitungen, bevorzugt kosmetische und dermatologische Zubereitungen, solche Substanzen enthaltend.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryontischen Einzellem. Sie können grob nach ihrer Form (Kugel, Zylinder, gekrümmter Zylinder) sowie nach dem Aufbau ihrer Zellwand (gram-positiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter freundii.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosol-sprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi [fungus = lat. Pilz], Mycota [µυκηζ = grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Ferner sind Superinfektionen der Haut durch Pilze und Bakterien nicht selten.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten - unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B. die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nennenswerte Einbußen erleidet.

Im Gegensatze zu den prokaryontischen und eukaryotischen zellulären Organismen sind Viren [virus = lat. Gift] biologische Strukturen, welche zur Biosynthese eine Wirtszelle benötigen. Extrazelluläre Viren (auch "Virionen" genannt) bestehen aus einer ein- oder doppelsträngigen Nukleinsäuresequenz (DNS oder RNS) und einem Proteinmantel (dapsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picomaviridae), Hepadnaviren (z.B. Hepatitis-B, Familie der Hepadnaviridae).

Viruzide, also Viren abtötende Substanzen im eigentlichen Sinne gibt es nicht, da Viren nicht über eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen ist jedenfalls schwierig. Mögliche Wirkmechanismen im Kampfe gegen die Viren sind in erster Linie die Störung deren Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie "antiviral" oder "gegen Viren wirksam", "viruzid" oder ähnlichen die Eigenschaft einer Substanz verstanden, einen ein- oder mehrzelligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Dem Stande der Technik mangelt es jedoch an gegen Viren wirksamen Substanzen, welche zudem den Wirtsorganismus nicht oder nicht in vertretbarem Maße schädigen.

Eine Aufgabe der vorliegenden Erfindung war also, diesem Übelstande abzuhelfen, also Substanzen zu finden, welche wirksam einen ein- oder mehrzelligen Organismus vor schädlichen Folgen einer Virusinfektion, sei es prophylaktisch oder therapeutisch, zu schützen.

Konservierungsmittel sind antimikrobielle Substanzen, die beim Herstellungsprozeß einem Produkt (Nahrungs- oder Genußmittel, pharmazeutische, kosmetische oder auch chemisch-technische Zubereitungen) in geringen Mengen (gewöhnlich je nach Produkt zwischen ca. 0,0005 % und 1 % Aktivgehalt) zugesetzt werden. Konservierungsmittel sollen Produkte während der Herstellung, der Lagerung und des Gebrauchs vor Verunreinigungen durch Mikroorganismen insbesondere vor den mikrobiell bedingten nachteiligen Veränderungen schützen.

An ein Konservierungsmittel werden grundsätzlich nachfolgende Forderungen gestellt: Es muß ausreichend antimikrobiell wirksam, technologisch anwendbar und gesundheitlich unbedenklich sein. Die gesundheitliche Unbedenklichkeit muß aber auch die fertige Zubereitung, das Handelsprodukt erfüllen. Dabei ist zu berücksichtigen, daß Mikroorganismen in z.B. kosmetischen Mitteln primär produktionsbedingt vorhanden sein oder sekundär durch den Verbraucher in das kosmetische Mittel gelangen können.

Daher muß gewährleistet sein, daß das Fertigprodukt auch über den gesamten Verbrauchszeitraum sicher ist.

Die meisten für eine Konservierung vorgeschlagenen bzw. vorgesehenen Konservierungsmittel wirken bakteriostatisch und fungistatisch, gelegentlich auch bakterizid und fungizid: sie sollen geruch- und geschmacklos und in den zur Anwendung kommenden Dosen nach Möglichkeit löslich, nicht toxisch, hautverträglich und ausreichend wirksam sein. Die Konservierungsmittel müssen, um wirksam zu sein, in dem zu konservierenden Roh- oder Hilfsstoff gelöst sein. Da die meisten Konservierungsmittel besser fett- als wasserlöslich sind, muß damit gerechnet werden, daß z. B. in einer Emulsion, deren wäßrige Phase konserviert werden soll, das in die wäßrige Phase eingearbeitete Konservierungsmittel im Verlauf der Lagerung in die Fettphase auswandert und damit die Konservierung der wäßrigen Phase in Frage gestellt ist. Aus diesem Grunde empfiehlt es sich, eine Kombination von Konservierungsmitteln einzusetzen, d. h. die wäßrige Phase mit einem gut wasserlöslichen Konservierungsmittel, die Fettphase dagegen gleichzeitig mit einem fettlöslichen Konservierungsmittel zu konservieren.

Für ein kosmetisches Präparat braucht zwar in der Regel keine Sterilität gefordert zu werden, es muß aber frei von pathogenen Keimen sein und vor mikrobiell bedingten Veränderungen geschützt werden.

Man sollte berücksichtigen, daß die verschiedensten Emulsionstypen, wäßrige Lösungen, Suspensionen usw. eine unterschiedliche Konservierung brauchen, daß die konservierende Wirkung einzelner Konservierungsmittel von der Zusammensetzung und den physikalischen Eigenschaften der zu konservierenden Zubereitung abhängig ist, daß mit Interaktionen zwischen dem Konservierungsmittel, den Wirk- und Hilfsstoffen zu rechnen ist, daß verschiedene Wirk- oder Hilfsstoffe Konservierungsmittel adsorbieren und damit möglicherweise inaktivieren können, daß insbesondere in der Zubereitung enthaltene Hydrokolloide konzentrationsabhängig Konservierungsmittel in ihrer antimikrobiellen Aktivität behindern können und daß schließlich - wiederum in Abhängigkeit von der Konzentration und dem Typ des Konservierungsmittels - das Stratum comeum das Konservierungsmittel adsorbiert und das Konservierungsmittel dann möglicherweise zur Permeation und Absorption kommt.

In der Lebensmitteltechnologie zugelassene Konservierungsmittel sind mit ihrer E-Nummer nachfolgend aufgeführt.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | -Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind in der Kosmetik gebräuchliche Konservierungsmittel oder Konservierungshilfsstoffe Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol.

4-Hydroxybenzoesäureester (Paraben-Ester, PHB-Ester), werden in freier Form oder als Natrium-Salze zur Konservierung von Fisch- und Fleischerzeugnissen, Mayonnaisen, Salatsaucen, kosmet. Präparaten, Arzneimitteln usw. verwendet.

Die als Konservierungsmittel wichtigsten 4-Hydroxybenzoesäureester sind 4-Hydroxybenzoesäuremethylester, 4-Hydroxybenzoesäureethylester, 4-Hydroxybenzoesäurepropylester, 4-Hydroxybenzoesäurebutylester. Der Methylester wurde auch als Sexuallockstoff läufiger Hündinnen identifiziert.

Die oft nur Methyl-, Ethyl-, Propyl-, Butyl- und Isobutylparaben genannten 4-Hydroxybenzoesäureester sind in Alkohol und Ether löslich, in Alkalilaugen lösen sie sich unter Salzbildung. Die Wirkung der Ester ist direkt proportional zur Kettenlänge des Alkyl-Restes, umgekehrt nimmt jedoch die Löslichkeit mit steigender Kettenlänge ab. Als nichtdissoziierende Verbindungen sind die Ester weitgehend pH-Wert-unabhängig und wirken in einem pH-Bereich von 3,0-8,0. Der antimikrobielle Wirkmechanismus beruht auf einer Schädigung der Mikrobenmembranen durch die Oberflächenaktivität der PHB-Ester sowie auf der Eiweiß-Denaturierung. Daneben treten Interaktionen mit Coenzymen auf. Die Wirkung richtet sich gegen Pilze, Hefen und Bakterien. Da PHB-Ester bereits ab einer Konzentration von ca. 0,08% in den Lebensmitteln zu geschmacklichen Beeinträchtigungen führen, ist die Verwendung in Lebensmitteln stark rückläufig.

Es wurde überraschend gefunden, und darin liegt die Lösung dieser Aufgabe, daß Wirkstoffkombinationen aus 3-lodo-2-propinyl-butylcarbamat und einem oder mehreren 4-Hydroxybenzoesäureestem sowie die Verwendung solcher Wirkstoffkombinationen als antibakterielle, antimycotische oder antivirale Wirkstoffe, den Nachteilen des Standes der Technik abhilft.

3-lodo-2-propinyl-butylcarbamat ist gekennzeichnet durch die chemische Strukturformel

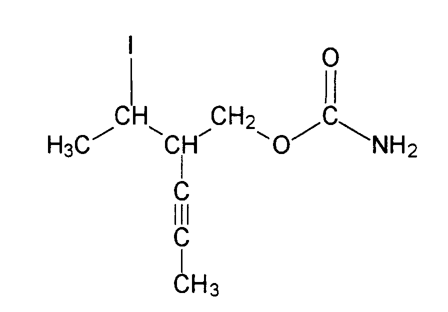

Ganz besonders vorteilhaft ist die Verwendung der erfindungsgemäßen Wirkstoffkombinationen als gegen grampositive Bakterien wirksame Wirkstoffe.

Es hat sich in erstaunlicher Weise herausgestellt, daß die erfindungsgemäß verwendeten Wirkstoffkombinationen, und zwar in überadditiver, also synergistischer Weise, das Wachstum von grampositiven und gramnegativen Bakterien, Mycobionten sowie Viren verhindern.

Insbesondere sind die erfindungsgemäß verwendeten Wirkstoffkombinationen befähigt, daß Wachstum von Hefen, insbesondere der Pityrosporum-Arten, namentlich Pityrosporum ovale, zu verhindern.

Es hat sich ferner herausgestellt, daß die erfindungsgemäß verwendeten Wirkstoffkombinationen die Bildung von seborrhoischen Erscheinungen, insbesondere Kopfschuppen, verhindern sowie bereits vorhandene seborrhoische Erscheinungen, insbesondere Kopfschuppen, zu beseitigen.

Die erfindungsgemäß verwendeten Wirkstoffkombinationen eignen sich darüber hinaus gut für die Verwendung als desodorierender Wirkstoff in kosmetischen Desodorantien sowie gegen unreine Haut, leichte Formen der Akne bzw. Propionibakterium acnes.

Die erfindungsgemäßen Wirkstoffkombinationen haben sich ebenfalls als besonders wirkungsvoll gegen Streptokokken erwiesen.

Schließlich hat sich herausgestellt, daß die erfindungsgemäß verwendeten Wirkstoffkombinationen den Verderb organischer Substanz, insbesondere kosmetischer und dermatologischer Zubereitungen, durch den Befall mit grampositiven und gramnegativen Bakterien, Mycobionten und Viren verhindern können, wenn sie diesen Zubereitungen zugesetzt werden.

Erfindungsgemäß sind somit auch ein Verfahren zur Bekämpfung von Mycobionten, dadurch gekennzeichnet, daß die erfindungsgemäß verwendeten Wirkstoffkombinationen gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, daß diesen organischen Produkten die erfindungsgemäß verwendeten Wirkstoffkombinationen in wirksamer Menge zugegeben werden.

Der Stand der Technik lieferte folglich nicht den geringsten Hinweis auf die erfindungsgemäße Verwendung als antimycotisches Wirkprinzip.

Ferner war erstaunlich, daß die erfindungsgemäß verwendeten Wirkstoffkombinationen besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Antischuppenshampoos.

Ganz besonders vorteilhaft im Sinne der vorliegenden Erfindung ist die Verwendung der erfindungsgemäßen Wirkstoffkombinationen zur Verhinderung des Verderbs organischer Substanz, insbesondere kosmetischer und dermatologischer Zubereitungen, durch den Befall mit grampositiven und gramnegativen Bakterien, Mycobionten und Viren.

Erfindungsgemäß bevorzugte 4-Hydroxybenzoesäureester sind 4-Hydroxybenzoesäuremethylester, 4-Hydroxybenzoesäureethylester, 4-Hydroxybenzoesäurepropylester, 4-Hydroxybenzoesäureisopropylester, 4-Hydroxybenzoesäurebutylester, 4-Hydroxybenzoesäureisobutylester.

Erfindungsgemäß werden die Wirkstoffkombinationen bevorzugt in kosmetischen oder dermatologischen Zusammensetzungen eingesetzt einem Gehalt von 0,0005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an den erfindungsgemäß verwendeten Wirkstoffen, ganz besonders vorteilhaft 0,5 - 3,0 Gew.-%, jeweils bezogen auf das Gesamtgewicht der Zusammensetzung.

Die erfindungsgemäß verwendeten Wirkstoffkombinationen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z.B. Nagellacke, Nagellackentferner, Nagelbalsame) und dergleichen.

Es ist auch möglich und gegebenenfalls vorteilhaft, die erfindungsgemäß verwendeten Wirkstoffkombinationen mit anderen Wirkstoffen zu kombinieren, beispielsweise mit anderen antimikrobiell, antimycotisch bzw. antiviral wirksamen Stoffen.

Werden die erfindungsgemäßen Wirkstoffkombinationen als Wirkstoffe zur Konservierung organischen Materials eingesetzt, so können auch vorteilhaft zusätzlich ein anderes oder mehrere andere Konservierungsmittel gewählt aus den Substanzen mit den E-Nummern von E-200 bis E-299 eingesetzt werden und/oder ein anderes oder mehrere andere Konservierungsmittel gewählt aus den Substanzen Dibromdicyanobutan (2-Brom-2-brom-methylglutarodinitril), Phenoxyethanol, 3-lod-2-propinylbutylcarbamat, 2-Brom-2-nitropropan-1,3-diol, Imidazolidinylharnstoff, 5-Chlor-2-methyl-4-isothiazolin-3-on, 2-Chloracetamid, Benzalkoniumchlorid, Benzylalkohol.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,5 zu wählen.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -mono- butylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- 2,4,6-Trianilino-(p-carbo-2'-ethyl-1'-hexyloxy)-1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entsprechenden 10-Sulfato-verbindungen, beispielsweise das entsprechende Natrium-, Kalium- oder Triethanolammonium-Salz), auch als Benzol-1,4-di(2-oxo-3-bomylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Es können die für die UVB-Kombination verwendeten Mengen eingesetzt werden.

Erfindungsgemäße Zubereitungen können, zumal wenn kristalline oder mikrokristalline Festkörper, beispielsweise anorganische Mikropigmente in die erfindungsgemäßen Zubereitungen eingearbeitet werden sollen, auch anionische, nichtionische und/oder amphotere Tenside enthalten. Tenside sind amphiphile Stoffe, die organische, unpolare Substanzen in Wasser lösen können.

Bei den hydrophilen Anteilen eines Tensidmoleküls handelt es sich meist um polare funktionelle Gruppen, beispielweise -COO', -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quatemären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

RNH₂⁺CH₂CH₂COOH X⁻ (bei pH=2) X⁻ = beliebiges Anion, z.B. Cl⁻

RNH₂⁺CH₂CH₂COO⁻ (bei pH=7)

RNHCH₂CH₂COO⁻ B⁺ (bei pH=12) B⁺ = beliebiges Kation, z.B. Na⁺

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind
Acylaminosäuren (und deren Salze), wie
   1. Acylglutamate, beispielsweise Natriumacylglutamat, Di-TEA-palmitoylaspartat und Natrium Caprylic/ Capric Glutamat,
   2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
   3. Sarcosinate, beispielsweise Myristoyl Sarcosin, TEA-lauroyl Sarcosinat, Natriumlauroylsarcosinat und Natriumcocoylsarkosinat,
   4. Taurate, beispielsweise Natriumlauroyltaurat und Natriummethylcocoyltaurat,
   5. Acyllactylate, lauroyllactylat, Caproyllactylat
   6. Alaninate
Carbonsäuren und Derivate, wie
   1. Carbonsäuren, beispielsweise Laurinsäure, Aluminiumstearat, Magnesiumalkanolat und Zinkundecylenat,
   2. Ester-Carbonsäuren, beispielsweise Calciumstearoyllactylat, Laureth-6 Citrat und Natrium PEG-4 Lauramidcarboxylat,
   3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie

Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 1 und 95 Gew.-% in den erfindungsgemäßen Zubereitungen vorliegen, bezogen auf das Gesamtgewicht der Zubereitungen.

Die Lipidphase der erfindungsgemäßen kosmetischen oder dermatologischen Emulsionen kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (z.B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -mono- butylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäße, als Emulsionen vorliegende Zubereitungen enthalten einen oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel

   R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH

   und n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19), Polyethylenglycol(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat
Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Die folgenden Beispiele sollen die Erfindung erläutern, aber nicht einschränken. Die Zahlenangaben beziehen sich auf Gew.-%, sofern nichts Anderes angegeben ist.

| **Beispiel 1** | |
|---|---|
| W/O-Lotion | |
| | Gew.-% |
| PEG-30 Dipolyhydroxystearat | 5,00 |
| Laurylmethiconcopolymer | 1,20 |
| Magnesiumstearat | 0,05 |
| Butylenglykol | 5,00 |
| Ceresin | 1,00 |
| Magnesiumsulfat | 0,50 |
| Butylparaben | 0,10 |
| Parfum | q.s. |
| Isohexadekan | 7,0 |
| Capric/Caprylic Triglyceride | 5,0 |
| Cetearylisononanoat | 14,0 |
| 3-lodo-2-propinyl-butylcarbamat | 0,10 |
| Wasser, demin. | ad 100,00 |

| **Beispiel 2** | |
|---|---|
| W/O-Lotion | |
| | Gew.-% |
| Polyglyceryl-2-dipolyhydroxystearat | 3,00 |
| Polyglyceryl-3-diisostearat | 2,00 |
| Kokosfettsäureglyceride | 3,00 |
| Glycerin | 3,00 |
| Ceresin | 0,50 |
| Magnesiumsulfat | 0,70 |
| 3-Iodo-2-propinyl-butylcarbamat | 0,05 |
| Propylparaben | 0,05 |
| Phenoxyethanol | 0,30 |
| Parfum | q.s. |
| Mineralöl | 12,0 |
| Dicaprylylether | 8,0 |
| Cetearylisononanoat | 6,0 |
| Wasser, demin. | ad 100.00 |

| **Beispiel 3** | |
|---|---|
| W/O-Lotion | |
| | Gew.-% |
| Polyglyceryl-2-dipolyhydroxystearat | 1,00 |
| Polyglyceryl-3-diisostearat | 1,00 |
| PEG-30 Dipolyhydroxystearat | 5,00 |
| DC Q2-5200 | 1,20 |
| Magnesiumstearat | 0,05 |
| Butylenglykol | 5,00 |
| Paraffin | 1,00 |
| Magnesiumsulfat | 0,50 |
| Methylparaben | 0,12 |
| Parfum | q.s. |
| Isohexadekan | 7,00 |
| Capric/Caprylic Triglyceride | 5,00 |
| Cetearylisononanoat | 14,00 |
| 3-lodo-2-propinyl-butylcarbamat | 0,100 |
| Wasser, demin. | ad 100,00 |

| **Beispiel 4** | |
|---|---|
| O/W-Lotion | |
| | Gew.-% |
| Glycerylstearat SE | 3,50 |
| Stearinsäure | 1,80 |
| Cyclomethicon | 3,00 |
| Cetylstearylalkohol | 0,50 |
| Natriumhydroxid (45%ig) | 0,20 |
| 3-lodo-2-propinyl-butylcarbamat | 0,03 |
| Isobutylparaben | 0,09 |
| Phenoxyethanol | 0,50 |
| Parfum | q.s. |
| Octyldodecanol | 7,0 |
| Dicaprylylether | 8,0 |
| Fucogel 1000 | 5,0 |
| Carbomer | 0,20 |
| Wasser, demin. | ad 100,00 |

| **Beispiel 5** | |
|---|---|
| Hydrodispersionsgel | |
| | Gew.-% |
| Pemulen TR-1 | 0,50 |
| Ethanol | 3,50 |
| Cyclomethicon | 3,00 |
| Dimethicon | 1,50 |
| Natriumhydroxid (45%ig) | 0,55 |
| 3-lodo-2-propinyl-butylcarbamat | 0,15 |
| Octyldodecanol | 0,50 |
| Capric/Caprylic Triglyceride | 5,00 |
| Methylparaben | 0,05 |
| Carbomer | 0,20 |
| Wasser, demin. | ad 100,00 |

| **Beispiel 6** | |
|---|---|
| O/W-Creme | |
| | Gew.-% |
| Glycerylstearat SE | 3,50 |
| Stearinsäure | 3,50 |
| Octyldodecanol | 5,00 |
| Cetylstearylalkohol | 3,00 |
| Natriumhydroxid (45%ig) | 0,35 |
| 3-lodo-2-propinyl-butylcarbamat | 0,10 |
| Parfum | q.s. |
| C₁₂-C₁₅ Alkylbenzoate | 10,0 |
| Butylparaben | 0,06 |
| Phenoxyethanol | 0,22 |
| Carbomer | 0,20 |
| Wasser, demin. | ad 100,00 |

| **Beispiel 7** | |
|---|---|
| Hydrodispersionsgel | |
| | Gew.-% |
| Pemulen TR-1 | 0,50 |
| Ethanol | 3,50 |
| Cyclomethicon | 3,00 |
| Dimethicon | 1,50 |
| Natriumhydroxid (45%ig) | 0,55 |
| 3-lodo-2-propinyl-butylcarbamat | 0,01 |
| Parfum | q.s. |
| Octyldodecanol | 0,50 |
| Capric/Caprylic Triglyceride | 5,00 |
| Methylparaben | 0,20 |
| Propylparaben | 0,10 |
| Carbomer | 0,20 |
| Wasser, demin. | ad 100,00 |

## Patentansprüche

1. Wirkstoffkombinationen aus 3-lodo-2-propinyl-butylcarbamat und einem oder mehreren 4-Hydroxybenzoesäureestem.

2. Verwendung von Wirkstoffkombinationen aus 3-lodo-2-propinyl-butylcarbamat und einem oder mehreren 4-Hydroxybenzoesäureestem als antibakterielle, antimycotische oder antivirale Wirkstoffe.

3. Wirkstoffkombinationen nach Anspruch 1 oder Verwendung nach Anspruch 2, **dadurch gekennzeichnet, daß** als 4-Hydroxybenzoesäureester eine oder mehrere Substanzen, aus der Gruppe 4-Hydroxybenzoesäuremethylester, 4-Hydroxybenzoesäureethylester, 4-Hydroxybenzoesäurepropylester, 4-Hydroxybenzoesäureisopropylester, 4-Hydroxybenzoesäurebutylester, 4-Hydroxybenzoesäureisobutylester gewählt wird oder werden.

4. Kosmetischen oder dermatologische Zusammensetzungen, enthaltend Wirkstoffkombinationen nach Anspruch 1, in Konzentrationen von 0,0005 - 50,0 Gew.-%, insbesondere 0,01 - 20,0 Gew.-%, bezogen auf das Gesamtgewicht der Zusammensetzung, bevorzugt 0,02 - 10,0 Gew.-%, besonders bevorzugt 0,02 - 5,0 Gew.-%, ganz besonders vorteilhaft 0,5 - 3,0 Gew.-%, jeweils bezogen auf das Gesamtgewicht der Zusammensetzung.

5. Verwendung nach Anspruch 2, zur Bekämpfung von seborrhoischen Erscheinungen, insbesondere Kopfschuppen.

6. Verwendung nach Anspruch 2 in kosmetischen Desodorantien oder Zubereitungen sowie gegen unreine Haut, leichte Formen der Akne bzw. Propionibakterium acnes.

7. Verwendung nach Anspruch 2, zur Verhinderung des Verderbs organischer Substanz, insbesondere kosmetischer und dermatologischer Zubereitungen, durch den Befall mit grampositiven und gramnegativen Bakterien, Mycobionten und Viren.
